# EUROPÄISCHE PATENTSCHRIFT

(11) **EP 1 083 848 B1**
(45) Veröffentlichungstag und Bekanntmachung des Hinweises auf die Patenterteilung: **15.10.2003**
(21) Anmeldenummer: 99927884.9
(22) Anmeldetag: 04.06.1999
(51) Int. Cl.: A61F 13/02

(54) **SELBSTKLEBENDE BANDAGE**
SELF-ADHESIVE BANDAGE
BANDAGE AUTO-ADHESIF

(30) Priorität: 09.06.1998 DE 19825577
(43) Veröffentlichungstag der Anmeldung: 21.03.2001
(73) Patentinhaber: Beiersdorf Aktiengesellschaft, 20245 Hamburg (DE)
(72) Erfinder: BODENSCHATZ, Stefan, D-21614 Buxtehude (DE); ANDREWS, Arthur-Hugh, D-25337 Kölln-Reisiek (DE); HARMAN, Anthony, David, Hertfordshire SG8 6EE (GB)
(86) Internationale Anmeldenummer: EP9903890
(87) Internationale Veröffentlichungsnummer: WO99063919

(56) Entgegenhaltungen:
- EP-A- 0 453 413
- DE-A- 2 517 790
- DE-A- 4 208 683
- US-A- 2 096 564

## Beschreibung

Die Erfindung betrifft eine selbstklebend beschichtete Bandage zur Stabilisierung und Fixierung von Gelenken und Extremitäten.

Bei Frakturen von Gelenken oder Extremitäten kommt klassischerweise ein Gipsverband zum Zuge, um die Stabilisierung beziehungsweise Fixierung des betroffenen Körperteils zu gewährleisten. Durch den Gipsverband wird die Bewegungsfähigkeit soweit eingeschränkt, daß das Knochengewebe wieder zusammenwachsen kann.

Weiterhin können Gipsverbände auch bei Rapturen von Bändern in Gelenken eingesetzt werden. Auch in diesem Fall soll der Verband jedwede Belastung beziehungsweise Bewegung unterbinden.

Derartige Gipsverbände können aus Gipsbinden oder synthetischen, Gipsbinden auf Reaktionsharzbasis bestehen. Bei beiden Systemen erfolgt eine Aushärtung durch das Benetzen mit Wasser. Dadurch wird in Verbindung mit den in den Binden eingearbeiteten Verstärkungsmaterialien die Festigkeit erreicht.

Diese Methoden sind bekannt. Sie haben aber auch Nachteile für den Anwender und Patienten. Natürliche Gipsverbande sind relativ kostengünstig, dafür aber schwer und nur begrenzt haltbar. Synthetische Gipsbinden müssen mit Handschuhen angelegt werden. Das Aushärten der Verbände benötigt bis zu 30 Minuten. Die synthetischen Gipsbinden müssen sehr aufwendig verpackt sein, weil sie im Kontakt mit Luftfeuchte aushärten.

Verletzungen von Bändern in Gelenken kann man auch mit der funktionellen Verbandtechnik, das sogenannte Taping, entgegen treten. Die Verbandtechnik ist darüber hinaus eine Behandlungsmethode zur Prophylaxe von Verletzungen, Krankheiten und Veränderungen am Bewegungsapparat. Taping hat zum Ziel, die Kapsel-Band-Strukturen gezielt nachzubilden und dadurch eine selektive Unterstützung und Stabilisierung zu erreichen. Eine Immobilisation wird damit jedoch nicht erzielt, wie sie mit Gipsverbänden angestrebt wird.

Der eigentliche Tapeverband wird dabei streifenweise aus vorzugsweise unelastischen selbstklebenden Bändern, sogenannten Zügen, oder in Verbindung mit kurzzugelastischen selbstklebenden Bändern angelegt. Er schützt, stützt und enttastet gefährdete, geschädigte oder gestörte Anteile einer Funktionseinheit. Er erlaubt die funktionelle Belastung im schmerzfreien Bewegungsraum; verhindert aber extreme oder schmerzhafte Bewegungen.
Als Trägermaterialien haben sich insbesondere Vliese, Gewebe oder Gewirke bewährt, die mit einem druckempfindlichen Kleber beschichtet sind. Auch beim Anlegen mehrere Lagen bleiben diese Verbände weitgehend flexibel.

Eine zum Ruhigstellen eines Körperteils geeignete Bandage ist in der EP 0 352 095 B beschrieben. Die Bandage besteht aus einem Substrat, dessen Oberflächen mit einer härfbaren flüssigen Verbindung imprägniert sind. Auf den Oberflächen sind weiterhin Abdeckungen vorhanden, die für Wasser durchgängig sind. Bei den Abdeckungen handelt es sich bevorzugt µm einen Vlies- oder Gewebeträger, der u.a. eine Fluorverbindung oder ein Silikon enthält.

DE-A-2517790 und US-A-2096564 offenbaren selbstklebende Bandagen, bestehend aus einem flexiblen Träger mit einer auf einer Seite aufgetragenen selbstklebenden Beschichtung. Der Träger ist mit einer Vielzahl von Durchbrüchen versehen. Die aus diesen Durchbrüchen sich ergebenden einzelnen Segmente sind nicht über schmale, im Vergleich zu den Segmenten, Verbindungsstege verknüpft. Die Bandagen ermöglichen somit eine Bewegungsfreiheit der Gelenke.

Aufgabe der Erfindung ist es, eine Bandage zur Verfügung zu stellen, die aufgrund ihrer Ausgestaltung, ihres Materials und ihrer Eigenschaften zur Stabilisierung und Fixierung von Gelenken und Extremitäten geeignet ist.

Gelöst wird diese Aufgabe durch eine Bandage, wie sie in den Ansprüchen 1 und 7 dargelegt ist. Gegenstand der Unteransprüche sind dabei vorteilhafte Weiterbildungen der Bandage.

Demgemäß besteht die erfindungsgemäße selbstklebehde Bandage aus einem flexiblen Träger mit einer auf einer Seite aufgetragenen selbstklebenden Beschichtung, wobei der Träger mit einer Vielzahl von Durchbrüchen versehen ist, so daß sich einzelne Segmente ergeben, die über Verbindungsstege verknüpft sind.

Bevorzugt werden als Träger eine Folie aus Olefinen oder ein Schaum aus Polyurethan verwendet.

In einer bevorzugten Ausführungsform weisen die Segmente eine konvexe Form auf. Auf diese Weise ist gewährleistet, daß bei der Erstellung eines Verbandes aus der Bandage ein verbesserter Kontakt zwischen den Segmenten in den einzelnen Lagen des Verbandes hergestellt wird.

In einer weiteren bevorzugten Ausführungsform weisen die Segmente eine regelmäßig geformte Kontur auf, insbesondere die Form von Sechsecken. In einer bevorzugten Ausführungsform haben die Sechsecke eine Weite von 7 mm und sind in einem Abstand von 0,5 mm auf dem Träger aufgebracht.
Zur Gewichtsersparnis können die Segmente darüber hinaus auch Ausnehmungen haben, die bevorzugt kreisförmig sind. Des weiteren können die Segmente aus geschäumten Material bestehen und/oder mit hohlen Kugeln oder Fasern abgemischt sein.
Die Segmente haben im beispielsweise eine Dicke von 0,5 bis 2 mm, insbesondere 1,5 mm.

Bevorzugt werden als Ausgangsmaterialien für die Segmente Kunststoffe gewählt wie PET, PP, PE und andere Polymere, so auch, wenn gewünscht, PVC. Aber auch PU (geschäumt/ungeschäumt) ist möglich, des weiteren Chitin und/oder Chitosan Schließlich sind auch mehrlagige Laminate geeignet, beispielsweise ein Laminat aus Pappe/Faserplatte/mit Aminoharzen versetztes Papier.

Bei Chitin (von griechisch χη 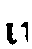 τον (chiton) Panzer) handelt es sich um ein besonders aus tierischen Organismen isoliertes Aminozucker-haltiges Polysaccharid der allgemeinen Formel (C₈H₁₃NO₅)ₓ, MR ca. 400 000. Chitin besteht aus Ketten von b-1,4-glykosidisch verknüpften N-Acetyl-D-glucosamin-(NAG-)Resten.

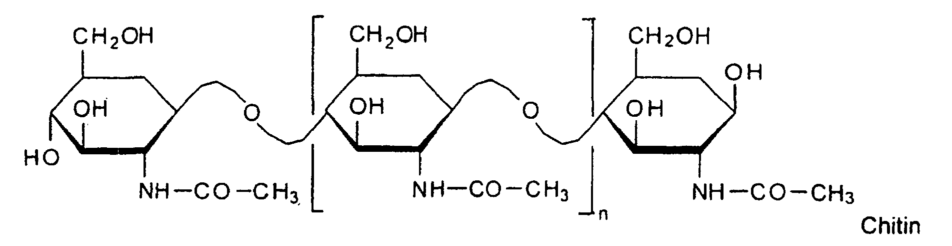

In Wasser, organischen Lösemitteln und verdünnten Laugen bzw. Säuren ist Chitin unlöslich. Starke Säuren spalten Chitin in D-Glucosamin (Chitosamin) und Essigsäure; bei Zerlegung durch Alkalien entstehen Acetate und das schwach basische, desacetylierte und teilweise depolymerisierte, kristallisierbare Chitosan, das in verdünnten Säuren (außer Schwefelsäure), wäßrigen Methanol und Glycerin löslich und außerdem gel- und filmbildend ist (siehe hierzu auch Römpp Lexikon Chemie, 10. Auflage, Stichwort "Chitin", Stuttgart/New York: Georg Thieme Verlag 1997).

Gefertigt werden können die Segmente, indem sie aus extrudierten Folien geschnitten oder gestanzt werden. Alternativ ist die Herstellung durch entsprechend dickes Aufdrucken möglich.

Die Segmente können auch die Form einer Pyramide oder eines Stumpfkegels sowie weiterer dreidimensionaler Körper aufweisen, die durch entsprechende Verbindungsstege miteinander verknüpft sind, so daß sich ein zusammenhängender Träger ergibt.
Als vorteilhaft hat sich herausgestellt, wenn der Träger dann eine Dicke von 0,3 bis 1 mm, insbesondere 0,5 mm aufweist
Die Fertigung kann erfolgen, indem die Segmente sowie die Verbindungsstege aus einer einzigen extrudierten Schicht geformt werden, so zum Beispiel durch das Schneiden mittels rotierender Messer oder mittels eines Wasserstrahls. Auch das Heißpressen ist möglich.

Die Bandage ist auf der Seite, die auf die Haut aufgelegt wird, mit einer der bekannten gut haftenden Selbstklebemassen auf, Basis von Kautschuk oder synthetischen Polymeren beschichtet. Vorteilhaft weisen die Massen weitere Eigenschaften wie gute Hautverträglichkeit oder Luft- und Wasserdampfdurchlässigkeit auf.

Als besonders vorteilhaft hat sich weiterhin erwiesen wenn die selbstklebende Beschichtung auf dem Träger eine Heißschmelzklebemasse mit einer Aktivierungstemperatur kleiner 70 °C ist, insbesondere 50 °C bis 60 °C.

Die selbstklebende Beschichtung weist insbesondere Dicken um 50 µm auf.

Des weiteren kann unterhalb der Klebebeschichtung auch eine Haftvermittler aufgebracht sein.

In einer weiteren bevorzugten Ausführungsform liegt die Breite der Bandage zwischen 4 und 15 cm. Die Dicke der Bandage sollte bevorzugt in einem Bereich von 1 bis 3 mm sein.

Die Klebeschicht kann bis zum Gebrauch der Bandage mit einem klebstoffabweisend ausgerüstetem Blattmateriäl wie beispielsweise silikonisiertem Papier oder Folie aus Kunststoff abgedeckt sein.

In einer weiteren bevorzugten Ausfuhrungsform besteht die selbstklebende Bandage, aus einem flexiblen Träger mit einer beidseitig aufgetragenen selbstklebenden Beschichtung, wobei der Träger mit einer Vielzahl von Durchbrüchen versehen ist, so daß sich einzelne Segmente ergeben, die über Verbindungsstege- verknüpft sind, wobei der Träger einseitig mit einem Hilfsträger eingedeckt ist.
Die Verbindungsstege können bei dieser Ausführungsform auch vollständig entfallen.

Der Hilfsträger besteht vorzugsweise aus einem in Längsrichtung nicht elastischen Gewebe oder Gewirke, das gegebenenfalls eine geringe Querelastizität aufweisen kann, insbesondere auf Baumwollbasis.

Insgesamt ergibt sich eine in Längsrichtung. nicht elastische Bandage, die aber in Querrichtung flexibel sein kann.

In einer vorteilhaften Anwendung der erfindungsgemäßen Bandage zur Stabilisierung und Fixierung von Gelenken und Extremitäten werden unterhalb der um die Gelenke und/oder Extremitäten gewickelten Bandage Schienen, komprimierbare Kissen und/oder Gewebe oder Vliese auf der Haut aufgelegt.

Die Vorteile der Bandage gegenüber den bisher bekannten liegen darin, daß die Bandage zur Bildung eines festen Verbandes eingesetzt werden kann, der vergleichbare Eigenschaften wie die bekannten Gipsverbände aufweist.
Durch das Wickeln der Bandage in mehreren Lagen entsteht ein sehr fester, das betroffene Gelenk beziehungsweise die betroffene Extremität in ihrer Bewegungsfähigkeit-stark einschränkender Verband.
Die Segmente legen sich beim Wickeln fischschuppenartig übereinander und werden miteinander verklebt. Durch die schmalen Verbindungsstege ist die Bandage gut anformbar, weil sich die Segmente einer Lage gegeneinander verschieben können. Es ist durch möglich und teilweise erwünscht, wenn beim Wickeln Stege brechen, so daß eine weitere Flexibilität erreicht wird. Durch die fischschuppenartige Struktur wird nach dem Anwickeln und Verkleben der einzelne Lagen eine hohe Stabilität erreicht, obwohl der sich sich ergebende Verband sehr viel leichter ist als herkömmlicher Naturgips.

Insbesondere bei der Beschichtung der Bandage beziehungsweise der Segmente mit einer Heißschmelzselbstklebemasse kann der Effekt erheblich gesteigert werden. denn durch kurzzeitiges Erwärmen des Verbandes und damit der Segmente kommt es zur Verklebung der Segmente auf der darunter befindlichen Lage der Bandage, so daß sich ein absolut inflexibler, mehrlagiger Verband ergibt.
Angestrebt wird, die notwendige Härte des Verbandes in ungefähr 15 Minuten zu erreichen, wie diese zwischen 50% und 90% des endgültigen Härte ausmacht.
Diese Festigkeit erstreckt sich dabei auch über einen Zeitraum von mehreren Wochen, wie er zur Abheilung der gängigen Verletzungen erforderlich ist.

Schließlich steht die Bandage durch die Wahl entsprechender Ausgangsmaterialien auch einem Röntgen nicht entgegen.

Schließlich kann die Bandage auch erneut verwendet werden, nachdem sie vom betroffenen Körperteil abgenommen worden ist.

Anhand der nachfolgend beschriebenen Figuren wird eine vorteilhafte Ausführung der Bandage näher erläutert, ohne damit die Erfindung unnötig einschränken zu wollen. Es zeigen
- Figur 1: einen Ausschnitt aus der erfindungsgemäßen Bandage,
- Figur 2: die Bandage in seitlichen Schnitt,
- Figur 3: einen Ausschnitt aus einem mehrtagigen Verband, der mit der erfindungsgemäßen Bandage gebildet worden ist und
- Figur 4: die Bandage mit einem Hilfsträger und ohne Verbihdunggstege.

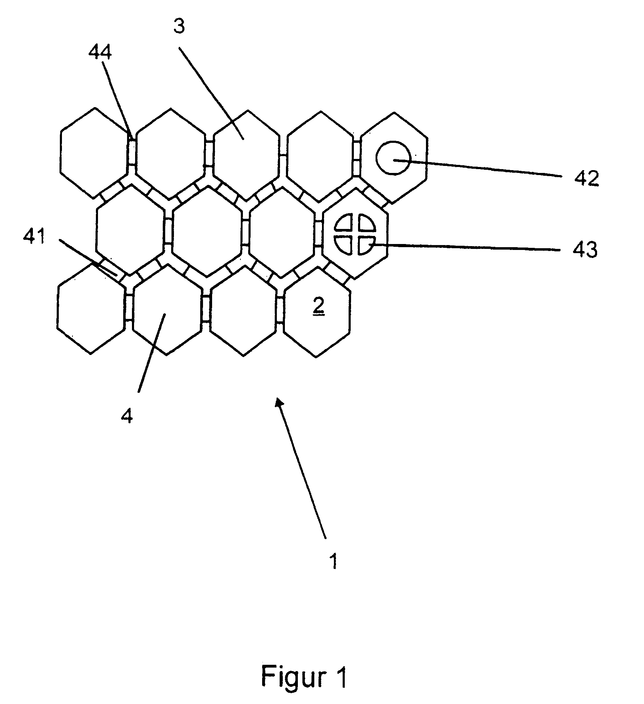

In der Figur 1 ist ein Ausschnitt aus der erfindungsgemäßen Bandage 1 dargestellt. Die Bandage 1 besteht aus einem flexiblen Träger 2, auf den einseitig eine selbstklebende Beschichtung 3 aufgetragen ist, und zwar eine Heißschmelzselbstklebemasse. Der Träger weist eine Vielzahl von Durchbrüchen 41 auf, so daß sich einzelne Segmente 4 ergeben, die wiederum über Verbindungsstege 44 miteinander verknüpft sind.

Die Segmente 4 sind in einem Muster angeordnet, wobei die Segmente 4 die Form von regelmäßigen Sechsecken aufweisen.

Zur Gewichtsersparnis können die Segmente 4 auch in unterschiedlicher Form mit Ausschnitten 42, 43 versehen sein, so können die Ausschnitte 42, 43 kreisförmig oder bevorzugt kreisabschnittsförmig sein.

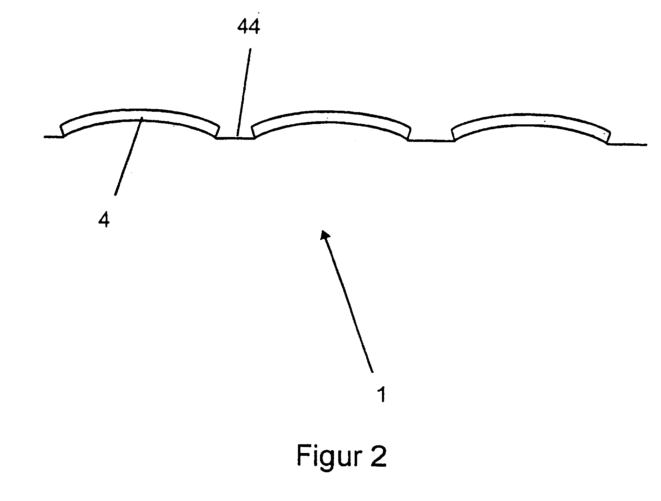

In der Figur 2 ist die Bandage 1 im seitlichen Schnitt dargestellt.
Die Segmente 4 weisen eine konvexe Form auf, so daß beim späteren Wickeln eines Verbandes 5 mit der Bandage 1 der Kontakt zwischen den einzelnen Segmenten 4 verbessert wird.

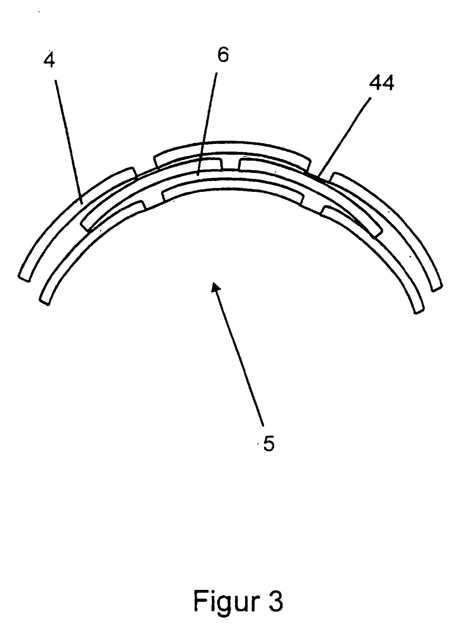

Ein derartiger Verband 5 ist ausschnittsweise in Figur 3 gezeigt. Durch das mehrlagige Wickeln des Verbandes 5 kommt es zu Überschneidungen der Segmente 4, so daß sich ein sehr stabiler Verband 5 um das zu behandelnde Körperteil ergibt. Die Stabilität wird dabei dadurch erhöht, daß der Verband 5 nach dem Anlegen kurzzeitig erwärmt wird, und zwar beispielsweise mit einem Heißluftfön. Es kommt zum Anschmelzen der Heißschmelzklebemasse, wobei sich deren Klebkraft erheblich verstärkt. Nach dem Erkalten sind die einzelnen Segmente 4 mit der jeweils darüber befindlichen Lage der Bandage 1 verbunden, es entsteht ein fester Verband 5.
Zwischen den einzelnen segmente 4 und der darunter befindlichen Lage der Bandage bilden sich Klebstoffbrücken 6 aus, die zur Verfestigung des Verbandes 5 führen.
Ein Ablösen des Verbandes 5 erfolgt, indem der Verband 5 erneut erwärmt wird, was zu einer Reduzierung der Klebkraft führt.

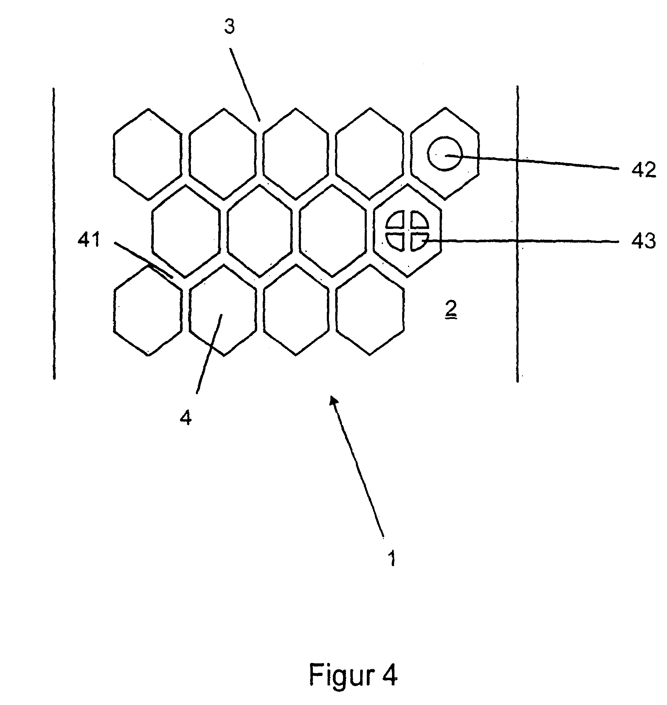

In der Figur 4 ist schließlich ein weiterer Ausschnitt aus der erfindungsgemäßen Bandage 1 dargestellt. Die Bandage 1 besteht aus einem flexiblen Träger 2, auf den beidseitig eine selbstklebende Beschichtung 3 aufgetragen ist, und zwar eine Heißschmelzselbstklebemasse.
Der Träger weist eine Vielzahl von Durchbrüchen 41 auf, so daß sich einzelne Segmente 4 ergeben, die hier allerdings nicht über Verbindungsstege 44 miteinander verknüpft sind.
Vielmehr wird die Stabilität der Bandage 1 durch einen Hilfsträger 21 gewährleistet. Die einzelnen Segmente 4 kleben auf dem Hilfsträger 21.

Die Segmente 4 sind in dem aus Figur 1 bekannten Muster angeordnet.

Zur Gewichtsersparnis können die Segmente 4 auch hier in unterschiedlicher Form mit Ausschnitten 42, 43 versehen sein, so können die Ausschnitte 42, 43 kreisförmig oder bevorzugt kreisabschnittsförmig sein.

## Patentansprüche

1. Selbstklebende Bandage, bestehend aus einem flexiblen Träger (2) mit einer auf einer Seite aufgetragenen selbstklebenden Beschichtung (3), **dadurch gekennzeichnet, dass** der Träger (2) mit einer Vielzahl von Durchbrüchen (41) versehen ist, so sich einzelne Segmente (4) ergeben, die über, im Verhältnis zu den Segmenten, schmale Verbindungsstege (44) verknüpft sind..

2. Selbstklebende Bandage gemäß Anspruch 1, **dadurch gekennzeichnet, daß** als Träger (2) eine Folie aus Olefinen oder ein Schaum aus Polyurethan verwendet werden.

3. Selbstklebende Bandage gemäß Anspruch 1, **dadurch gekennzeichnet, daß** die Segmente (4) eine konvexe Form aufweisen.

4. Selbstklebende Bandage gemäß Anspruch 1, **dadurch gekennzeichnet, daß**, die Segmente (4) eine regelmäßig geformte Kontur aufweisen, insbesondere die Form von Sechsecken, und gegebenenfalls Ausnehmungen (42, 43) haben.

5. Selbstklebende Bandage gemäß zumindest einem der vorhergehenden Ansprüche, **dadurch gekennzeichnet, daß** die selbstklebende Beschichtung (3) des Trägers (2) eine Heißschmelzklebemasse mit einer Aktivierungstemperatur kleiner 70 °C ist, insbesondere 50°C bis 60 °C.

6. Selbstklebende Bandage gemäß Anspruch 1, **dadurch gekennzeichnet, daß** die Breite der Bandage zwischen 4 und 15 cm liegt.

7. Selbstklebende Bandage, bestehend aus einem flexiblen Träger (2) mit einer beidseitig aufgetragenen selbstklebenden Beschichtung (3), **dadurch gekennzeichnet, dass** der Träger (2) mit einer Vielzahl von Durchbrüchen (41) versehen ist, so dass sich einzelne Segmente (4) ergeben, die über, im Verhältnis zu den Segmenten, schmale Verbindungsstege (44) verknüpft sind, wobei der Träger (2) einseitig mit einem Hilfsträger (21) eingedeckt ist.

8. Selbstklebende Bandage gemäß zumindest einem der vorhergehenden Ansprüche, **dadurch gekennzeichnet, daß** die Bandage auf ihrer selbstklebenden Seite mit klebstoffabweisendem Material abgedeckt ist.

9. Verwendung der Bandage gemäß zumindest einem der vorhergehenden Ansprüche zur Stabilisierung und Fixierung von Gelenken und Extremitäten, wobei unterhalb der um die Gelenke und/oder Extremitäten gewickelten Bandage Schienen, komprimierbare Kissen und/oder Gewebe oder Vliese auf der Haut aufgelegt werden.

## Claims

1. Self-adhesive bandage, consisting of a flexible support (2) with a self-adhesive coating (3) applied on one side, **characterized in that** the support (2) is provided with a large number of apertures (41), thus giving rise to individual segments (4) linked by way of connecting bridges (44) which are narrow in relation to the segments.

2. Self-adhesive bandage according to Claim 1, **characterized in that** a film made of olefins or a foam made of polyurethane is used as support (2).

3. Self-adhesive bandage according to Claim 1, **characterized in that** the segments (4) have a convex shape.

4. Self-adhesive bandage according to Claim 1, **characterized in that** the segments (4) have a regularly shaped contour, in particular the shape of hexagons, and optionally have recesses (42, 43).

5. Self-adhesive bandage according to at least one of the preceding claims, **characterized in that** the self-adhesive coating (3) of the support (2) is a hot-melt adhesive composition with an activation temperature of less than 70°C, in particular of 50°C to 60°C.

6. Self-adhesive bandage according to Claim 1, **characterized in that** the width of the bandage is between 4 and 15 cm.

7. Self-adhesive bandage, consisting of a flexible support (2) with a self-adhesive coating (3) applied on both sides, **characterized in that** the support (2) is provided with a large number of apertures (41), thus giving rise to individual segments (4) linked by way of connecting bridges (44) which are narrow in relation to the segments, said support (2) being covered on one side with an auxiliary support (21) .

8. Self-adhesive bandage according to at least one of the preceding claims, **characterized in that** the bandage is covered, on its self-adhesive side, with anti-adhesive material.

9. Use of the bandage according to at least one of the preceding claims for stabilizing and fixing joints and extremities, with splints, compressible pads and/or wovens or nonwovens being placed on the skin below the bandage which is wound around the joints and/or extremities.

## Revendications

1. Bandage auto-adhésif, se composant d'un support flexible (2) avec un revêtement (3) auto-adhésif appliqué d'un côté, **caractérisé en ce que** le support (2) est pourvu d'une pluralité d'interruptions (41) de sorte qu'il en résulte des segments individuels (4), qui sont reliés par des languettes de liaison (44) qui sont étroites par comparaison avec les segments.

2. Bandage auto-adhésif selon la revendication 1, **caractérisé en ce que** l'on utilise comme support (2) une feuille d'oléfines ou une mousse de polyuréthanne.

3. Bandage auto-adhésif selon la revendication 1, **caractérisé en ce que** les segments (4) présentent une forme convexe.

4. Bandage auto-adhésif selon la revendication 1, **caractérisé en ce que** les segments (4) présentent un contour de forme régulière, notamment la forme d'hexagones, et présentent éventuellement des évidements (42, 43).

5. Bandage auto-adhésif selon au moins l'une des revendications précédentes, **caractérisé en ce que** le revêtement auto-adhésif (3) du support (2) est une composition adhésive thermofusible avec une température d'activation inférieure à 70°C, en particulier de 50°C à 60°C.

6. Bandage auto-adhésif selon la revendication 1, **caractérisé en ce que** la largeur du bandage est comprise entre 4 et 15 cm.

7. Bandage auto-adhésif, se composant d'un support flexible (2) avec un revêtement (3) auto-adhésif appliqué des deux côtés, **caractérisé en ce que** le support (2) est pourvu d'une pluralité d'interruptions (41) de sorte qu'il en résulte des segments individuels (4), qui sont reliés par des languettes de liaison (44) qui sont étroites par comparaison avec les segments, le support (2) étant recouvert d'un côté par un support auxiliaire (21) .

8. Bandage auto-adhésif selon au moins l'une des revendications précédentes, **caractérisé en ce que** le bandage est recouvert sur son côté auto-adhésif par un matériau anti-adhésif.

9. Utilisation du bandage selon au moins l'une des revendications précédentes, pour la stabilisation et la fixation d'articulations et d'extrémités, dans lequel, en dessous du bandage enroulé autour de l'articulation et/ou des extrémités, on place sur la peau des attelles, des coussins comprimables et/ou des tissus ou des voiles.
